# EUROPEAN PATENT SPECIFICATION

(11) **EP 3 833 963 B1**
(45) Date of publication and mention of the grant of the patent: **02.08.2023**
(21) Application number: 19765563.2
(22) Date of filing: 08.08.2019
(51) Int. Cl.: G01N 21/552, G01N 31/22, G01N 21/78, G01N 33/02, B82Y 30/00, G01N 33/12

(54) **A COLORIMETRIC METHOD FOR THE DETECTION OF ORGANIC MERCURY**
KOLORIMETRISCHES VERFAHREN ZUM NACHWEIS VON ORGANISCHEM QUECKSILBER
PROCÉDÉ COLORIMÉTRIQUE DE DÉTECTION DE MERCURE ORGANIQUE

(30) Priority: 10.08.2018 IT 201800008034
(43) Date of publication of application: 16.06.2021
(73) Proprietor: Fondazione Istituto Italiano di Tecnologia, 16163 Genova (IT); UNIVERSITA' DEGLI STUDI DI GENOVA, 16126 Genova (IT)
(72) Inventor: DONATI, Paolo, 63900 Fermo (IT); POMPA, Pier Paolo, 16163 Genova (IT)
(74) Representative: Comoglio, Elena
(86) International application number: PCT/IB2019/056765
(87) International publication number: WO 2020/031134

(56) References cited:
- LIU YI ET AL: "Highly selective, colorimetric detection of Hg2+based on three color changes of AuNPs solution from red through sandy beige to celandine green", SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, ELSEVIER BV, NL, vol. 249, 21 April 2017 (2017-04-21), pages 331-338, XP085031953, ISSN: 0925-4005, DOI: 10.1016/J.SNB.2017.04.116
- TORU MATSUI ET AL: "Accurate Standard Hydrogen Electrode Potential and Applications to the Redox Potentials of Vitamin C and NAD/NADH", JOURNAL OF PHYSICAL CHEMISTRY. A, MOLECULES, SPECTROSCOPY,KINETICS, ENVIRONMENT AND GENERAL THEORY, vol. 119, no. 2, 5 January 2015 (2015-01-05), pages 369-376, XP055554341, US ISSN: 1089-5639, DOI: 10.1021/jp508308y
- JIN LI-HUA ET AL: "Eco-friendly colorimetric detection of mercury(II) ions using label-free anisotropic nanogolds in ascorbic acid solution", SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, vol. 195, 20 January 2014 (2014-01-20), pages 239-245, XP028626539, ISSN: 0925-4005, DOI: 10.1016/J.SNB.2014.01.020
- LI WU ET AL: "Chemical Vapor Generation for Determination of Mercury by Inductively Coupled Plasma Mass Spectrometry", APPLIED SPECTROSCOPY REVIEWS., vol. 42, no. 2, 1 March 2007 (2007-03-01), pages 79-102, XP055554357, US ISSN: 0570-4928, DOI: 10.1080/05704920601184234
- CHENG-YAN LIN ET AL: "Colorimetric Sensing of Silver(I) and Mercury(II) Ions Based on an Assembly of Tween 20-Stabilized Gold Nanoparticles", ANALYTICAL CHEMISTRY, vol. 82, no. 16, 15 August 2010 (2010-08-15), pages 6830-6837, XP055553838, US ISSN: 0003-2700, DOI: 10.1021/ac1007909
- See Xing ET AL: "A simple visual and highly selective colorimetric detection of Hg 2+ based on gold nanoparticles modified by 8-hydroxyquinolines and oxalates", Chem. Commun. Chem. Commun, 1 January 2014 (2014-01-01), pages 6447-6447, XP55626939, Retrieved from the Internet: URL:https://pubs.rsc.org/en/content/articl ehtml/2014/cc/c4cc00069b [retrieved on 2019-09-27]
- PAOLO DONATI ET AL: "Nanocatalyst/Nanoplasmon-Enabled Detection of Organic Mercury: A One-Minute Visual Test", ANGEWANDTE CHEMIE, INTERNATIONAL EDITION, vol. 58, no. 30, 22 July 2019 (2019-07-22) , pages 10285-10289, XP55626859, DE ISSN: 1433-7851, DOI: 10.1002/anie.201905669

## Description

The invention relates to a colorimetric method for the detection of organic mercury.

In particular, the colorimetric method of the invention is applicable to the detection of organic mercury in complex matrices, such as for example food matrices.

Identifying and quantifying heavy metals in food matrices is an increasingly felt need, especially since several diseases, even with fatal outcomes, have been related to exposure to these substances.

Heavy metals - such as lead, mercury, cadmium, selenium - are characterised by high density (> 5.0 g/cm³), low solubility of their hydrates, ability to form complexes, and a certain affinity for sulphides. It is because of these chemical-physical characteristics that they can be carcinogenic and neurotoxic. In this context, mercury is particularly interesting given its high organ and systemic toxicity even at low concentrations.

Mercury naturally occurs in different forms and its organometallic compounds are notoriously the most harmful to human health. Among these, methylmercury (CH₃Hg, CH₃Hg⁺), which is formed from inorganic mercury by the action of anaerobic organisms present in aqueous ecosystems, can receive special mention. Due to its high liposolubility, methylmercury is bioaccumulated and biomagnified along the trophic chain. However, the high toxicity problem is not limited to methylmercury alone, but is brought about by all organometallic mercury compounds, which have the ability to accumulate in the central nervous system through L-amino acid transporters (LAT) after conjugation with the amino acid L-cysteine. This blocks protein synthesis and cell division in nerve cells, causing massive glutamate release and activation of N-methyl-D-aspartate (NMDA) receptors, thereby destroying calcium homeostasis.

The maximum limit of mercury in various food matrices is established by law. In drinking water, for example, the Environmental Protection Agency (EPA) has set the maximum level of the contaminant at 2 ppb, whereas the European Union has set this limit at 1 ppb.

Maximum methylmercury values have also been regulated in food. For example, the EU regulation No. 420/2011 of 29 April 2011 established a threshold of 0.5 ppm for food products (e.g., fish), whereas the FDA set the threshold at 1 ppm. Given the growing scientific evidence of the danger of methylmercury, in 2017 the EPA and the FDA issued a joint statement ("2017 EPA-FDA Advice"), which recommends to select the fish to be eaten based on the presumed content of methylmercury and to reduce the consumption of fish species with the highest methylmercury content.

Today, in order to identify and dose mercury it is necessary to use techniques that require expensive instrumentation, highly qualified personnel, and long and costly procedures. Universally recognized techniques are atomic absorption spectroscopy (AAS) or atomic emission spectroscopy (AES), and inductively coupled plasma mass spectrometry (ICP-MS).

Instead, it would be preferable to have methods which allow sample analysis and detection of toxic metals directly at the sampling site, quickly, and at low cost. Such methods would pave the way for numerous applications in scenarios ranging from the domestic environment to food production and distribution facilities, and to developing countries. For this reason, in recent years, there have been numerous attempts to develop innovative techniques for the detection of heavy metals and in particular organic mercury.

For example, techniques have been developed, which involve the use of chromogens capable of forming a coloured adduct with the target, or the use of other reagents whose binding to the target causes a chemical-physical change, such as a change in the pH^{4.5} (Lee, S. Y.; Lee, J. J.; Bok, K. H.; Kim, J. A.; So, Y. K.; Kim, C. A Colorimetric Chemosensor for the Sequential Recognition of Mercury (II) and Iodide in Aqueous Media. Inorg. Chem. Commun. 2016, 70, 147-152; Li, X.; Su, X.; Shi, Z.; Cheng, X.; Liu, S.; Zhao, Q. Highly Selective and Reversible Colori-metric Detection of Mercury Ions by a Hydrophilic Cycloruthenated Complex in Water. Sensors Actuators B Chem. 2014, 201, 343-350).

Other strategies exploit electron transfer reactions to produce a potential difference, colorimetric redox reactions, or the activation of fluorescent probes (Nazeeruddin, M. K.; Di Censo, D.; Humphry-Baker, R.; Grätzel, M. Highly Selective and Reversible Optical, Colorimetric, and Electrochemical Detection of Mercury(II) by Amphiphilic Ruthenium Complexes Anchored onto Mesoporous Oxide Films. Adv. Funct. Mater. 2006, 16 (2), 189-194; Lohani, C. R.; Neupane, L. N.; Kim, J.; Lee, K.-H. Selectively and Sensitively Monitoring Hg2+ in Aqueous Buffer Solutions with Fluorescent Sensors Based on Unnatural Amino Acids. Sensors Actuators B Chem. 2012, 161 (1), 1088-1096).

Further techniques are based on the use of metallic nanoparticles used as "probes". For example, the surface catalytic activity of noble metal nanoparticles can be exploited to amplify a response, often obtaining clear signals in a short time. Platinum, gold and palladium nanoparticles are known to have remarkable catalytic properties also in relation to the high surface/volume ratio. In fact, these nanoparticles are known as "artificial enzymes" or "nanozymes". The great advantage of these technologies is that, with appropriate engineering, it is possible to obtain "synthetic enzymes" in which selectivity, activity and stability far exceed those of protein analogues.

Several detection schemes known in the art are based on direct interaction between the analyte (cation) and the nanozyme, with the consequent passivation of the nanoparticle surface. The cation of interest, after having been reduced, settles on the surface of the nanoparticle, forming thereon a layer that blocks the catalytic activity thereof. For example, this strategy allows inorganic mercury to be discriminated from other cations, such as Pb²⁺, Cd²⁺, Ni²⁺, As³⁺ e Cr³⁺, thanks to their different standard reduction potential values. In control conditions, it is possible to selectively reduce the Hg²⁺ ion, since it has an E₀ value higher than that of the other metal cations (E₀ (Hg) = + 0.855 V).

This principle is the basis of the strategy described in Wu, G.-W.; He, S.-B.; Peng, H.-P.; Deng, H.-H.; Liu, A.-L.; Lin, X.-H.; Xia, X.-H.; Chen, W. Citrate-Capped Platinum Nanoparticle as a Smart Probe for Ultrasensitive Mercury Sensing, which exploits the formation of metallic mercury on the surface of platinum nanoparticles. In the presence of inorganic mercury (Hg²⁺), the peroxidase activity of platinum nanoparticles is inhibited in a concentration-dependent manner and detected by the formation of a coloured adduct starting from an oxidizable chromogen. Although this approach allows inorganic mercury to be measured *in situ* (in water samples), this method is not suitable for the detection of organic mercury (for example methylmercury), since the latter is not able to form a passivation layer on nanoparticles. In fact, the authors of the above article only analyse inorganic mercury. The same group of G.W. Wu and colleagues in patent application CN 104267026 A proposed a method for *in situ* detection of mercury (II).

Similarly, Peng, C.-F.; Zhang, Y.-Y.; Wang, L.-Y.; Jin, Z.-Y.; Shao, G. in Colorimetric Assay for the Simultaneous Detection of Hg 2+ and Ag + Based on Inhibiting the Peroxidase- like Activity of Core-Shell Au@Pt Nanoparticles describe a method based on the use of nanoparticles with a platinum-coated gold core, which allows the detection of the presence of inorganic mercury and silver cations, without however being able to discriminate them. In this case, too, the method does not allow the detection of organic mercury species. Moreover, the method does not allow a detection with the naked eye, but only allows an instrumental detection.

Chen, Z.; Wang, X.; Cheng, X.; Yang, W.; Wu, Y.; Fu, F. in Specifically and Visually Detect Methyl-Mercury and Ethyl-Mercury in Fish Sample Based on DNA-Templated Alloy Ag-Au Nanoparticles. Anal. Chem. 2018, 90 (8), 5489-5495 describe a method that exploits the different affinity of cations such as Ag⁺, Au⁺ e C₂H₅Hg⁺, CH₃Hg⁺ for thymine-rich aptamers. In the absence of the analyte, the gold and silver cations are linked to single-stranded DNA sequences. The addition of a reducing agent induces the formation of small-sized Au-Ag alloy nanoparticles on DNA strands, without colour development. In the presence of mercury compounds, these compete with the aptamer-bound cations, replacing them in order of affinity. In this case, the cations released in solution will form larger Ag-Au alloy nanoparticles with different plasmonic properties which give the solution a purple colour. The result is a colorimetric response due to the formation of different particles, with different optical properties, in relation to cation displacement phenomena. Although this method is able to detect organic mercury forms in solution, it has some limitations associated with the use of aptamers, especially in terms of stability of the reagents at room temperature.

Parallel to catalytic properties, other strategies exploit the quantum interaction with light radiation, whereby solutions of these particles change colour depending on the aggregation state or following surface changes. Often, this property is combined with surface functionalization with appropriate probes, such as amino acids or oligonucleotide aptamers, which specifically recognize certain targets, thereby obtaining a selective response. A limitation of these approaches is that the use of biomolecules, whether enzymes or aptamers, often involves the need to maintain salinity, pH and temperature values not too different from the physiological values, both to prevent enzyme denaturation and to guarantee the functioning of the mechanisms of recognition and interaction between the biomolecules and the targets.

For instance, in Aulsebrook, M. L.; Watkins, E.; Grace, M. R.; Graham, B.; Tuck, K. L. Modified Gold Nanoparticles for the Temperature-Dependent Colorimetric Detection of Mercury and Methylmercury. Chemis-trySelect 2018, 3 (7), 2088-2091, gold nanoparticles have been functionalized with a probe that has the amino acid thymine at its end, which by binding to the mercury target makes the nanoparticles aggregate into adducts with a different visible light absorption peak than that of the monodisperse nanoparticles. Depending on the analyte concentration in the sample, the solution exhibits a change in colour from red-pink to purple-blue. The limitation of this approach is that aggregation requires stoichiometric amounts of analyte and therefore the technique is not sensitive to concentrations comparable to the limits of the law.

Chen, L.; Li, J.; Chen, L. in Colorimetric Detection of Mercury Species Based on Functionalized Gold Nanoparticles. ACS Appl. Mater. Interfaces 2014, 6 (18), 15897-15904 describe a method based on the change in colour of a solution of gold nanoparticles. As mercury displays a much higher affinity to diethyldithiocarbamate (DDTC) compared to other cations, in the presence of mercury the latter is successful in replacing the Cu-DDTC bond, releasing copper ions in solution. The Hg-DDTC complex, through its thiol groups, then binds to the surface of the gold nanoparticles, causing them to aggregate. The main limitations of this method are the sensitivity and operation in terms of point-of-care.

Li et al. in Catalysis-reduction strategy for sensing inorganic and organic mercury based on gold nanoparticles. Biosensors and Bioelectronics 2017, Vol. 92, 15 June 2017, pages 328-334 propose a strategy for the detection of inorganic and organic mercury in an aqueous solution, which is based on the cooperative effect of the catalytic properties of gold nanoparticles (AuNPs) and the formation of an amalgam. AuNPs can catalyse HEPPSO into its catalytic product, which has an absorption peak at about 340 nm and is exploited as an indicator for the detection of mercury species. HEPPSO has been shown to be able to reduce mercury species (Hg²⁺, Hg⁺, CH₃Hg⁺) to Hg0. Hg0 shows high affinity to the surface of AuNPs, with amalgam formation. If several mercury species are present in the solution, Hg(I,II) can be reduced to Hg(°) by HEPPSO. Hg(°) then occupies the active catalytic sites on the surface of the nanoparticles forming an amalgam, which decreases the catalytic activity of the AuNPs, with a consequent decrease in the quantity of catalytic product, accompanied by a decrease in the intensity of the characteristic absorption peak at 340 nm. Lastly, when the entire surface of the AuNPs is covered by mercury atoms, the catalytic activity of the nanoparticles is completely inhibited, and the conversion of HEPPSO into its catalytic product ceases. The progressive change in the intensity of the absorption peak at 340 nm is observed by UV-VIS spectroscopy.

The paper of Liu Yi et al.: "Highly selective, colorimetric detection of Hg2+ based on three color changes of AuNPs solution from red through sandy beige to celandine green", Sensors and Actuators B, vol. 249, 21 April 2017, pages 331-338, discloses a method for the detection of Hg²⁺ ions in aqueous solution based on a color change of gold nanoparticles due to their aggregation in presence of Hg²⁺ with the assistance of ascorbic acid and a further aggregating agent (SH-HPEI).

The analytical/diagnostic platforms currently available and suitable for *in situ* detection of organomercurial compounds are therefore not many.

Developing a diagnostic strategy which allows the presence of organic mercury to be detected in a simple manner is therefore of great practical interest, in order to carry out frequent screenings in food samples or other complex matrices.

For this purpose, the present invention provides a colorimetric method for the detection of organic mercury compounds in a fluid to be analysed, as defined in appended claim 1.

The dependent claims, which define further advantageous features of the method of the invention, form an integral part of the present specification.

In the method of the invention, the change in colour from red to purple/blue is caused by the reduction of organic mercury to metallic mercury and the consequent aggregation of the gold nanoparticles. Therefore, in the absence of organic mercury, the gold nanoparticles do not aggregate and the fluid remains red.

In step 1 of the method defined by appended claim 1, the fluid to be analysed, for example, is a liquid phase extract from any animal or plant tissue or organic material. According to a preferred embodiment, the sample of fluid is a liquid phase extract from a food product.

According to a preferred embodiment, the gold nanoparticles are added to the sample of fluid to be analysed to a final concentration between 50 pM and 50 nM, as calculated based on the volume including the sample and reagents, preferably between 100 pM and 1 nM, expressed as the number of particles per litre.

The method of the invention advantageously allows the presence of organometallic mercury to be detected in any sample of fluid, even in liquid phase complex matrices, by exploiting a synergistic mechanism of reduction of organic mercury to metallic mercury, induced by the combined use of a reducing agent and the surface effect, which is related to the presence of low-energy nucleation sites on the surface of gold nanoparticles. The reducing agent promotes the redox reaction that reduces organic mercury into its metallic form. This reaction takes place on the surface of the gold nanoparticles, leading to the assisted deposition of metallic mercury on the surface of the nanoparticles, which are destabilized and aggregate. This causes the displacement of the plasmonic band of the gold nanoparticles, with a visible change in colour of the solution from red to purple/blue.

The synergistic mechanism described above shifts the balance of the reaction towards the products and therefore favours the reduction of the organometallic precursors. This synergy causes the reduction reaction to metallic mercury to be extremely rapid (1-2 minutes at room temperature), a crucial feature in order to exploit this process in a point-of-care colorimetric detection method. On the other hand, in the absence of a reducing agent, the reduction reaction of organic mercury to metallic mercury (Hg°) does not occur because in these conditions it is energetically unfavoured. Similarly, the required co-participation of the nanoparticles is also confirmed by the fact that in the absence thereof, the reduction of mercury into the metallic state is not significant because it is unfavoured.

Moreover, the method of the invention provides the use of mild reducing agents for the reduction of organic mercury, without compromising the speed and selectivity of the reaction, which is made possible thanks to the synergistic effect described above. This is extremely advantageous since the use of strong reducing agents, which are known to be toxic reagents, would prevent the use thereof in a point-of-care mode, i.e. outside the laboratory facility. Preferred mild reducing agents are formic acid and oxalic acid.

The mild reducing agent is added to the sample to be analysed in such a quantity that its final concentration is between 0.1 and 100 mM. The preferred concentration in the final solution is between 5 and 20 mM. The exact concentration of the mild reducing agent is selected according to the type of sample to be analysed (for example, drinking water or river water or complex matrix) and the selection thereof within the above-mentioned range falls within the skills of those skilled in the art.

The gold nanoparticles used in the method of the invention are particularly suitable as they have a high chemical-physical stability and a high extinction coefficient.

As previously mentioned, the method of the invention provides the use of mild reducing agents. These substances, compared to the conventionally used strong reducing agents, such as sodium borohydride (NaBH₄) or nitric acid, are less reactive and therefore also less aggressive. In fact, they are numbered among the biocompatible substances and, thanks to the absence of toxicity, they respect the green chemistry principles. Such reducing agents can be inorganic or organic. Oxalic acid and formic acid were shown to be particularly effective. Other suitable organic mild reducing agents are citric acid and acetaldehyde. Suitable inorganic mild reducing agents are hydroxylamine, phosphites and sulfites.

In addition to biocompatibility, the use of mild reducing agents also allows a further advantage, namely an increased specificity of the detection method. Stronger reducing agents, such as for example sodium borohydride, would in fact result in an extremely lower detection sensitivity. With strong reducing agents, in fact, mercury (Hg°) has a certain probability of nucleating in a more independent manner, i.e. it tends to form a metallic mercury nanoparticulate, without nucleating on the surface of the gold nanoparticles. This inhibits the nanoparticle aggregation phase and, consequently, significantly reduces the sensitivity of the method. With the use of mild reducing agents, on the other hand, the formation of the mercury nanoparticulate is clearly less and therefore the final sensitivity is higher.

Furthermore, the use of strong reducing agents would lead to a reduction in specificity, since other metals, such as for example cadmium and lead, would also nucleate on the surface of the gold nanoparticles, thus giving false positives. Instead, by using mild reducing agents, only mercury can be reduced and can nucleate on the gold nanoparticles.

The method of the invention also makes it possible to avoid the use of surface functionalization of accessory and excess nanoparticles.

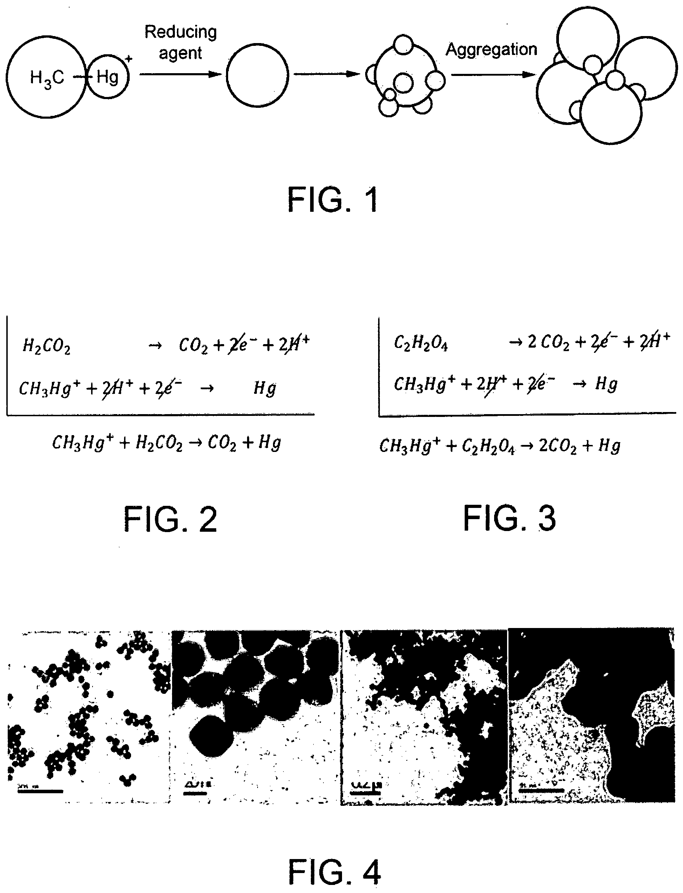

Figure 1 shows the reaction scheme of the method of the invention. The method consists of two synergistic steps: the reducing agent, by means of a redox reaction, allows the formation of mercury in the metallic state (Hg°), which settles on the surface of the nanoparticles. The nucleation of metallic mercury is energetically favoured by the availability of a substrate for which it has affinity, i.e. the gold nanoparticles, and this further favours reduction, shifting the redox reaction towards the products. Subsequently, the gold particles, destabilized by the nucleated mercury on their surface, aggregate giving rise to a change in colour of the solution from red to blue/purple. Thanks to this synergistic mechanism, which favours the reduction of organic mercury both kinetically and energetically, the reaction is extremely rapid and provides a clear colorimetric response, which is detectable by the naked eye.

Figures 2 and 3 show the specific redox reactions occurring between methylmercury - formic acid and methylmercury - oxalic acid; formic acid and oxalic acid, in fact, were shown to be the most effective mild reducing agents.

Below is a table showing the standard enthalpy of formation values (Δ_{f}H°) for various compounds belonging to the methylmercury and ethylmercury classes.

| Substance | Standard enthalpy of formation (ΔfH°) kJ mol-1 |
|---|---|
| Ethylmercury bromide | -107.5 |
| Ethylmercury chloride | -141.1 |
| Ethylmercury iodide | -65.7 |
| Methylmercury bromide | -86.2 |
| Methylmercury chloride | -116.3 |
| Methylmercury iodide | -43.5 |

Since the Δ_{f}H° values are very similar, the same stability, and therefore the same reactivity, is expected for the organometallic bonds in the two classes of compounds. Therefore, the method of the invention is applicable to the detection not only of methylmercury, but also of other organic mercury compounds, including particularly ethylmercury.

The examples that follow are provided for illustration purposes only and do not limit the scope of the invention, which is defined by the appended claims.

### EXAMPLES

### Example 1: Preparation of a tissue extract to be analysed (pre-treatment)

A sample of tissue to be analysed was prepared as follows:
1) weighing of a known quantity of tissue (in a range of 5 - 10 ± 0.05 g);
2) homogenization;
3) addition of a 10:1:1 mixture of MilliQ water: HCl/CH₃COOH:Cysteine;
4) mixing and removal of the sediment with a 0.2 mm Teflon filter or a mini column.

### Example 2: Detection of methylmercury

The tests below used a 0.1 mg/mL aqueous solution of methylmercury chloride, which was characterised by ICP. This solution was then diluted in water to obtain samples with known concentrations of methylmercury.

Gold nanoparticles with a diameter of 35 nm, obtained according to the procedure described in P.P. Pompa, G. Vecchio, A. Galeone, V. Brunetti, G. Maiorano, S. Sabella, and R. Cingolani "Physical assessment of toxicology at nanoscale: nano dose-metrics and toxicity factor" Nanoscale 3, 2889-97 (2011) were added to the samples at known concentrations of methylmercury (sample volume = 100 uL), in such a quantity as to obtain a final concentration of 150 pM of the gold nanoparticles in the sample. Subsequently, 10uL of a 50 mM solution in formic acid were added up to a final concentration of 5 mM in the sample; this induced aggregation, detectable with the naked eye, which was characterised spectrophotometrically. This concentration of the reducing agent allows the colour change to be detected within 2 minutes of reaction.

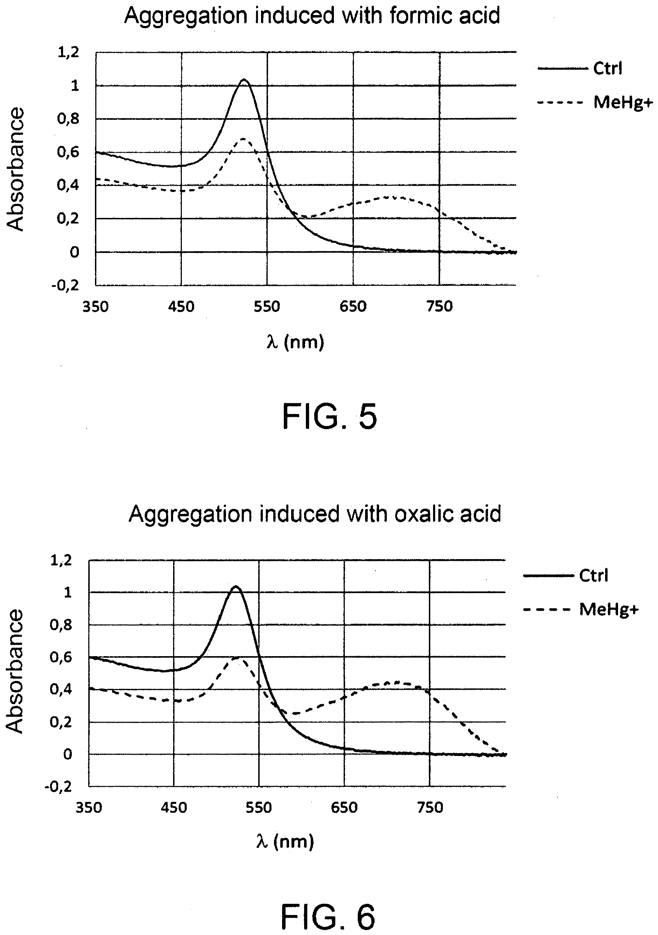

Figure 4 shows images, obtained by TEM microscopy, which describe the aggregation process. The first two pictures from the left show that the nanoparticles are monodisperse. This data is confirmed by the spectrum (Figures 5-6) with a peak around 520 nm. The third and fourth images from the left, instead, show that the particles have become aggregated once they have been covered by reduced mercury, such that the individual units are no longer recognizable. These formations, which have a much larger average size than the starting nanoparticles, display a plasmonic peak shifted to a different wavelength.

The graphs of Figure 5 and Figure 6 show the effects of the aggregation in terms of shift of the plasmonic peak when formic acid and oxalic acid, respectively, are used as mild reducing agents. The solid line curves represent the spectrum of the control samples (no methylmercury), whereas the dotted line curves refer to samples containing methylmercury.

In particular, Figure 5 shows the shift of the plasmonic peak following aggregation of the nanoparticles when formic acid was used as the mild reducing agent. Figure 6 shows the shift of the plasmonic peak of the system following aggregation of the nanoparticles when oxalic acid was used as the mild reducing agent.

It has been established that the method of the invention is sensitive to methylmercury concentrations of 1 µM. Considering that the legal limit currently in force for food matrices (fish) is 5 µM, the applicative utility of the method of the invention compared to other less sensitive methods known in the art is evident.

### Example 3: Comparative experiments

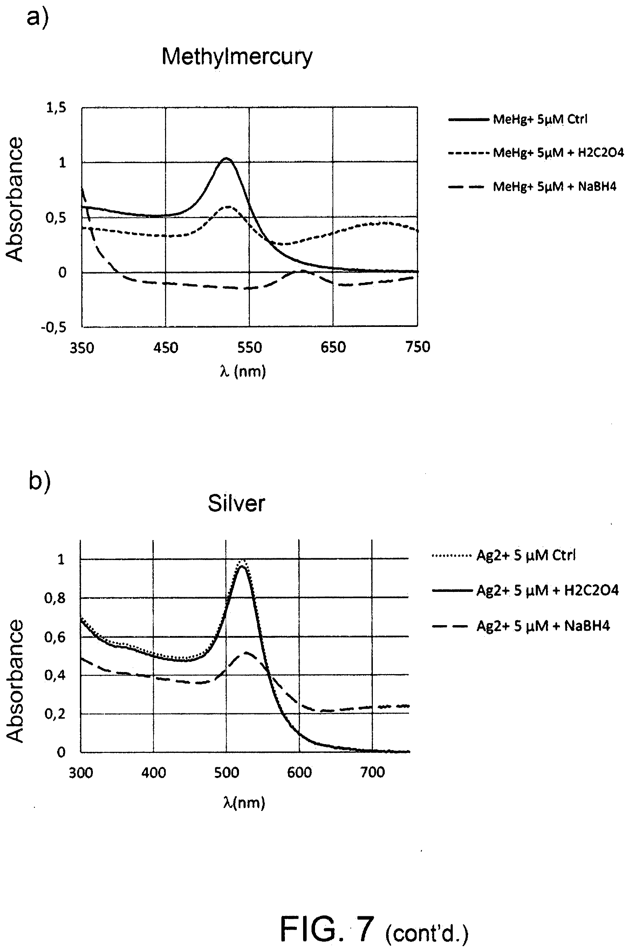

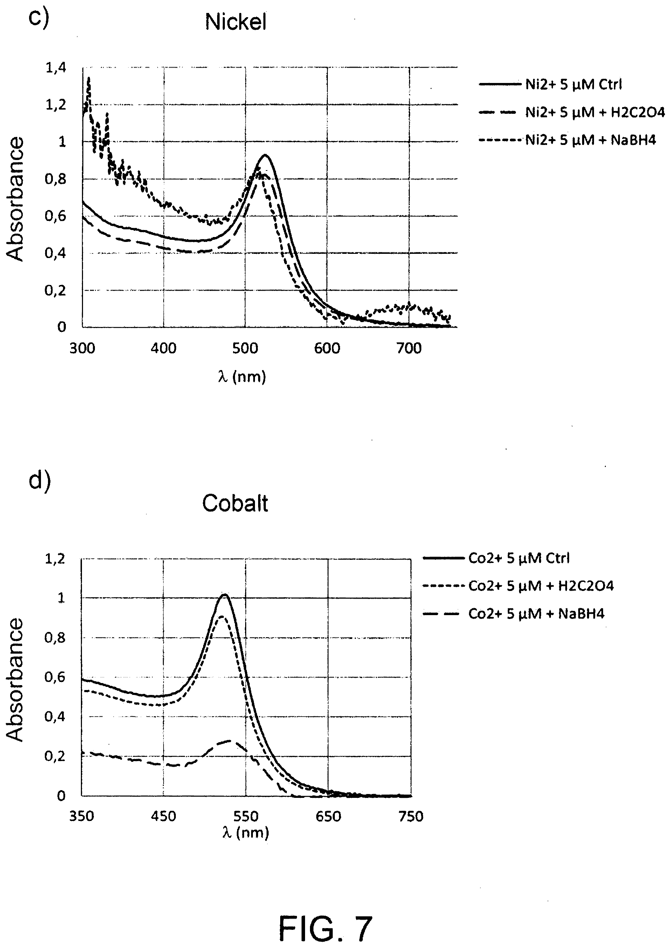

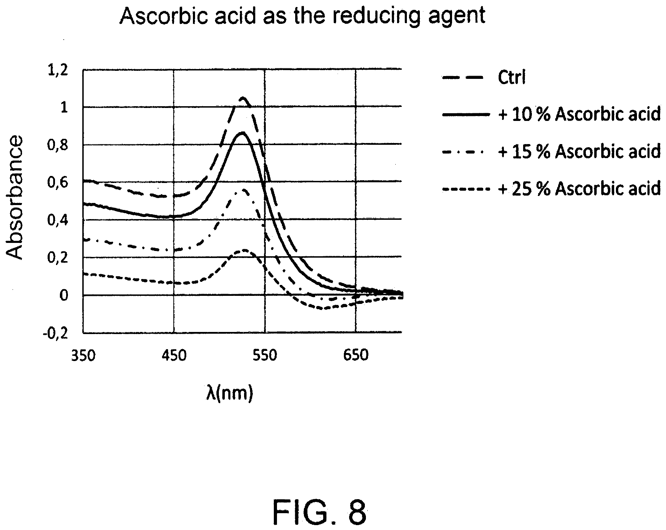

The present inventors carried out some comparative experiments, the results of which are described below with reference to Figures 7 and 8.

Figure 7a illustrates the results obtained in the detection of methylmercury by using, on the one hand, a mild reducing agent (i.e. formic acid), and on the other hand a much stronger reducing agent, that is sodium borohydride. In Figure 7a, the plasmonic spectrum of the methylmercury sample added with gold nanoparticles is represented by the solid line. The spectrum of the same sample added with formic acid is represented by the short-dashed line: this spectrum shows the absorption peak of the aggregate, with colour change of the solution indicative of the presence of the analyte. The spectrum represented by the long-dashed line, on the other hand, shows the effect of sodium borohydride which induces uncontrolled aggregation.

The same experiment was repeated with an aqueous solution of silver ions and the results are shown in Figure 7b. In this case, the addition of formic acid does not cause the sample to turn blue/purple; in contrast, the use of sodium borohydride gives a false positive as the particles are destabilized even in the absence of the analyte cation. Another drawback is the undesired formation of nanostructures starting from the same contaminant, if present at high concentrations. The formation of hydrogen gas from the decomposition of the reducing agent is also observed, which invalidates the reading and the detection with the naked eye.

The same experiment was also repeated on a sample with added nickel. The results are shown in Figure 7c. By using formic acid as the reducing agent, no aggregation or colour change of the solution is found, as can be seen from the long-dashed line spectrum. In contrast, when using sodium borohydride, the sample is destabilized and false positives are obtained, as can be seen from the short-dashed line spectrum.

Figure 7d shows the results of a similar experiment carried out with cobalt. In this case, too, by using formic acid, no aggregation or colour change of the solution is found (short-dashed line spectrum); by using sodium borohydride, on the other hand, a non-specific reaction (false positive) is obtained, illustrated by the long-dashed line spectrum, which lacks the plasmonic peak characteristic of the aggregation of gold particles.

The results illustrated in Figures 7a-d demonstrate the selectivity of the colorimetric method of the invention for the detection of organic mercury compounds.

Lastly, Figure 8 relates to the results obtained by using a reducing agent with a very low reduction potential, such as ascorbic acid. Ascorbic acid does not lead to reduction and consequently to deposition of the sought cation. By adding increasing amounts of ascorbic acid, the cation of interest cannot be reduced, but the gold nanoparticle system becomes unstable and false positives are generated due to non-specific aggregation, which occurs both in the presence of mercury ions and in the absence thereof. The graph in Figure 8 shows that following the addition of ascorbic acid (10-15%) to a 1µM solution of methylmercury (concentration close to the detection limit) the aggregation peak does not appear, whereas with high concentrations of the reducing agent (25-30%) the system is destabilized even in the absence of mercury.

## Claims

1. A colorimetric method for the detection of organic mercury compounds in a fluid to be analysed, consisting of the steps of:
1) providing a sample of the fluid to be analysed and adding thereto gold nanoparticles having a diameter between 2 and 200 nm;
2) adding thereto a mild reducing agent selected from the group consisting of oxalic acid, formic acid, citric acid, acetaldehyde, hydroxylamine, phosphites, sulfites, and any combination thereof;
3) detecting, by observation with the naked eye, the colour of the sample of fluid added with the gold nanoparticles and the reducing agent, the change in colour of said sample from red to purple/blue, caused by aggregation of the gold nanoparticles, being indicative of the presence of organic mercury compounds in the sample of fluid.

2. The method according to claim 1, wherein the fluid to be analysed is a liquid phase extract from any animal or plant tissue or organic material.

3. The method according to claim 2, wherein the fluid to be analysed is a liquid phase extract from a food product.

4. The method according to any one of claims 1 to 3, wherein said organic mercury compounds are methylmercury and/or ethylmercury.

5. The method according to any one of claims 1 to 4, wherein the gold nanoparticles have a diameter between 10 nm and 40 nm.

6. The method according to any one of claims 1 to 5, wherein the gold nanoparticles are added to the sample of fluid to be analysed to a final concentration between 50 pM and 50 nM, expressed as the number of nanoparticles per litre, preferably to a final concentration between 100 pM and 1 nM, expressed as the number of nanoparticles per litre.

7. The method according to any one of claims 1 to 6, wherein the mild reducing agent is added to the sample of fluid to be analysed to a final concentration between 0.1 mM and 100 mM, preferably to a final concentration between 5 mM and 20 mM.

8. The method according to any one of claims 1 to 7, which is carried out at room temperature.

## Patentansprüche

1. Kolorimetrisches Verfahren zum Nachweis organischer Quecksilberverbindungen in einer zu untersuchenden Flüssigkeit, aufweisend die folgenden Schritte:
1) Bereitstellen einer Probe der zu untersuchenden Flüssigkeit und Zugabe von Gold-Nanopartikeln mit einem Durchmesser zwischen 2 und 200 nm;
2) Zugabe eines schwachen Reduktionsmittels, ausgewählt aus der Gruppe bestehend aus Oxalsäure, Ameisensäure, Zitronensäure, Acetaldehyd, Hydroxylamin, Phosphiten, Sulfiten und jeglichen Kombinationen davon;
3) Bestimmen der Farbe der Probe der mit den Gold-Nanopartikeln versetzten Flüssigkeit und des Reduktionsmittels durch Beobachtung mit dem bloßen Auge, wobei die durch Aggregation der Gold-Nanopartikel hervorgerufene Farbveränderung der Probe von rot zu violett/blau das Vorhandensein organischer Quecksilberverbindungen in der Probe der Flüssigkeit anzeigt.

2. Verfahren nach Anspruch 1, wobei die zu untersuchende Flüssigkeit ein Flüssigphasenextrakt aus einem beliebigen tierischen oder pflanzlichen Gewebe oder organischem Material ist.

3. Verfahren nach Anspruch 2, wobei die zu untersuchende Flüssigkeit ein Flüssigphasenextrakt aus einem Lebensmittelerzeugnis ist.

4. Verfahren nach einem der Ansprüche 1 bis 3, wobei die organische(n) Quecksilberverbindungen) Methylquecksilber und/oder Ethylquecksilber ist/sind.

5. Verfahren nach einem der Ansprüche 1 bis 4, wobei die Gold-Nanopartikel einen Durchmesser zwischen 10 nm und 40 nm haben.

6. Verfahren nach einem der Ansprüche 1 bis 5, wobei die Gold-Nanopartikel der Probe der zu untersuchenden Flüssigkeit bis zu einer Endkonzentration zwischen 50 pM und 50 nM zugegeben werden, dargestellt als Zahl der Nanopartikel pro Liter, bevorzugt bis zu einer Endkonzentration zwischen 100 pM und 1 nM, dargestellt als Zahl der Nanopartikel pro Liter.

7. Verfahren nach einem der Ansprüche 1 bis 6, wobei das schwache Reduktionsmittel der Probe der zu untersuchenden Flüssigkeit bis zu einer Endkonzentration zwischen 0,1 mM und 100 mM, bevorzugt bis zu einer Endkonzentration zwischen 5 mM und 20 mM zugegeben wird.

8. Verfahren nach einem der Ansprüche 1 bis 7, welches bei Raumtemperatur durchgeführt wird.

## Revendications

1. Procédé colorimétrique pour la détection de composés organiques de mercure dans un fluide à analyser, comprenant les étapes consistant à :
1) fournir un échantillon du fluide à analyser et y ajouter des nanoparticules d'or ayant un diamètre entre 2 et 200 nm ;
2) y ajouter un agent réducteur doux sélectionné dans le groupe constitué de l'acide oxalique, de l'acide formique, de l'acide citrique, de l'acétaldéhyde, de l'hydroxylamine, des phosphites, des sulfites, et de n'importe quelle combinaison de ceux-ci ;
3) détecter, par observation à l'oeil nu, la couleur de l'échantillon de fluide auquel ont été ajoutés les nanoparticules d'or et l'agent réducteur, le changement de couleur dudit échantillon du rouge au violet/bleu, provoqué par l'agrégation des nanoparticules d'or, indiquant la présence de composés organiques de mercure dans l'échantillon de fluide.

2. Procédé selon la revendication 1, dans lequel le fluide à analyser est un extrait en phase liquide provenant n'importe quel tissu animal ou végétal ou d'une matière organique.

3. Procédé selon la revendication 2, dans lequel le fluide à analyser est un extrait en phase liquide d'un produit alimentaire.

4. Procédé selon l'une quelconque des revendications 1 à 3, dans lequel lesdits composés organiques de mercure sont le méthylmercure et/ou l'éthylmercure.

5. Procédé selon l'une quelconque des revendications 1 à 4, dans lequel les nanoparticules d'or ont un diamètre entre 10 nm et 40 nm.

6. Procédé selon l'une quelconque des revendications 1 à 5, dans lequel les nanoparticules d'or sont ajoutées à l'échantillon de fluide à analyser à une concentration finale entre 50 pM et 50 nM, exprimée en nombre de nanoparticules par litre, de préférence à une concentration finale entre 100 pM et 1 nM, exprimée en nombre de nanoparticules par litre.

7. Procédé selon l'une quelconque des revendications 1 à 6, dans lequel l'agent réducteur doux est ajouté à l'échantillon de fluide à analyser à une concentration finale entre 0,1 mM et 100 mM, de préférence à une concentration finale entre 5 mM et 20 mM.

8. Procédé selon l'une quelconque des revendications 1 à 7, qui est réalisé à température ambiante.
